(19) Europäisches Patentamt
European Patent Office
Office européen des brevets

(11) Publication number: **0 104 857**
**A1**

## (12) EUROPEAN PATENT APPLICATION

(21) Application number: 83305481.0

(22) Date of filing: 19.09.83

(51) Int. Cl.³: C 07 H 19/06
A 61 K 31/70

(30) Priority: 28.09.82 GB 8227643

(43) Date of publication of application:
04.04.84 Bulletin 84/14

(84) Designated Contracting States:
CH DE FR GB IT LI NL

(71) Applicant: BEECHAM GROUP PLC
Beecham House Great West Road
Brentford Middlesex(GB)

(72) Inventor: Harnden, Michael Raymond
47 Guildford Road
Horsham Sussex(GB)

(72) Inventor: Shanks, Colin Thomas
12 Meath Green Avenue
Horley Surrey(GB)

(72) Inventor: Luk, Kong
4 Palmer Close
Horley Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

## (54) Deoxyuridine compounds, methods for preparing them and their use in medicine.

(57) A compound of formula (I):

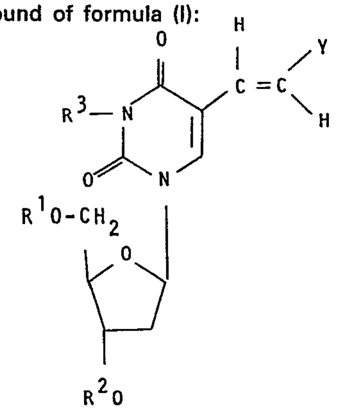

(I)

in which Y is chlorine, bromine or iodine, each of $R^1$ and $R^2$ is hydrogen or a group of formula

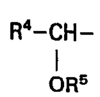

in which $R^4$ is hydrogen, optionally halo-substituted $C_{1-6}$ alkyl, phenyl or phenyl $-C_{1-6}$ alkyl, $R^5$ is optionally halo-substituted $C_{1-6}$ alkyl, or phenyl$-C_{1-6}$alkyl, or $R^4$ and $R^5$ complete a ring system and together represent $-(CH_2)_3-$, $-(CH_2)_4-$ or $-CH_2OCH_2CH_2-$, and $R^3$ is hydrogen or an acyl group of formula

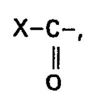

in which X is $C_{1-6}$alkyl, phenyl or phenyl$-C_{1-6}$alkyl, each of which is optionally substituted with halogen, with the proviso that $R^1$ and $R^2$ are not simultaneously hydrogen, is useful in treating viral infections.

EP 0 104 857 A1

Croydon Printing Company Ltd.

DEOXYURIDINE COMPOUNDS, METHODS FOR PREPARING THEM AND THEIR USE IN MEDICINE

This invention relates to certain deoxyuridine compounds which have antiviral activity.

Published European Patent Application No. 0 061 283 discloses esters of 5-(2-halogenovinyl)-2'-deoxyuridines which have anti-viral activity selective against herpes viruses.

We have now found a group of mono-, di- and tri-substituted 5-(2-halogenovinyl)-2'-deoxyuridines which have antiviral activity, and which are useful in the treatment of infections caused by herpes viruses, such as herpes simplex type 1, herpes simplex type 2 and varicella.

According to the present invention there is provided a compound of formula (I):

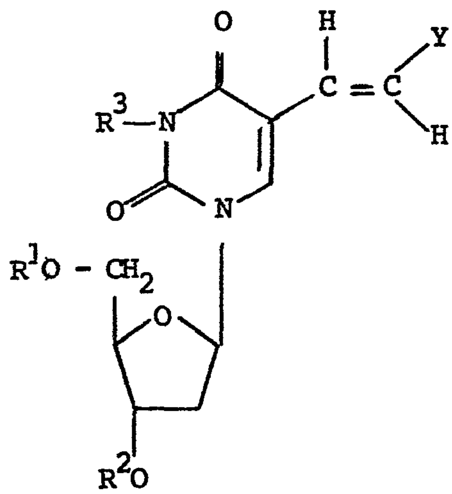

(I)

in which Y is chlorine, bromine or iodine, preferably bromine, each of $R^1$ and $R^2$ is hydrogen or a group of formula $R^4-\underset{OR^5}{\underset{|}{CH}}-$ in which $R^4$ is hydrogen, optionally halo-substituted $C_{1-6}$ alkyl, phenyl or phenyl - $C_{1-6}$ alkyl, $R^5$ is optionally halo-substituted $C_{1-6}$ alkyl or phenyl-$C_{1-6}$alkyl, or $R^4$ and $R^5$ complete a ring system and together represent $-(CH_2)_3-$, $-(CH_2)_4-$ or $-CH_2OCH_2CH_2-$, and $R^3$ is hydrogen or an acyl group of formula $X-\underset{O}{\underset{\|}{C}}-$, in which X is $C_{1-6}$alkyl, phenyl or phenyl-$C_{1-6}$alkyl, each of which is optionally substituted with halogen, with the proviso that $R^1$ and $R^2$ are not simultaneously hydrogen.

Preferably, $R^2$ is hydrogen.

Preferably, $R^4$ is hydrogen, methyl, chloro-methyl, ethyl or phenyl, and $R^5$ is methyl, ethyl or benzyl. When $R^4$ and $R^5$ together complete a ring system they preferably represent $-(CH_2)_4-$.

When $R^3$ is an acyl group, X is preferably optionally substituted phenyl, suitably chloro-substituted phenyl.

Compounds of formula (I) in which $R^4$ and $R^5$ complete a ring system, may be prepared by treating a compound of formula (II):

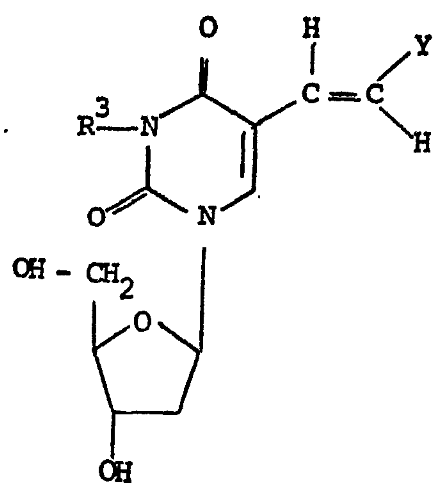

(II)

in which Y and $R^3$ are as defined in formula (I), with an ether of formula (III):

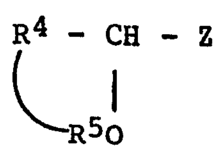

(III)

in which Z is hydrogen or a $C_{1-6}$ alkoxy group, preferably t-butoxy and, when Z is hydrogen, the $-R^4-R^5$ group includes a double bond adjacent to the oxygen.

The reaction is preferably carried out in an inert organic solvent, suitably tetrahydrofuran, and in the presence of an acid catalyst, suitably p-toluene sulphonic acid.

The reaction will generally produce a mixture of three compounds wherein substitution is present at (a) the 3' position, (b) the 5' position, and (c) the 3' and 5' positions. The mixture may be separated into its individual components by standard chromatographic methods.

Compounds of formula (I) in which $R^4$ and $R^5$ do not complete a ring system may be prepared by treating a compound of formula (II), as defined, with a compound of formula (IV):

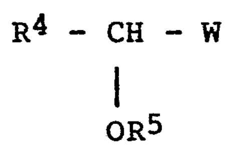

(IV)

in which W is a $C_{1-6}$alkoxy group or a halogen atom, preferably chlorine.

When W is $C_{1-6}$ alkoxy the reaction is preferably carried out in a similar reaction medium to that employed in the reaction between compounds of formulae (II) and (III), and a mixture of two compounds will

generally be produced in which substitution is present at (a) the 3' and 5' positions, and (b) the 5' position. As before, the mixture may be separated into its components by chromatographic methods.

When W is halogen, the reaction is preferably carried out in an inert solvent, suitably dimethylformamide, in the presence of a basic catalyst, suitably diisopropylethylamine. This reaction is particularly suitable for preparing compounds of formula (I) wherein $R^5$ represents a phenyl-$C_{1-6}$alkyl group and $R^4$ represents hydrogen. A similar reaction, using 2,4-lutidine as the basic catalyst, is particularly suitable for preparing compounds of formula (I) wherein $R^5$ represents $C_{1-6}$ alkyl and $R^4$ represents hydrogen. Again, a mixture of 3',5' disubstituted and 3' or 5' mono-substituted compounds will generally be produced and the individual compounds can be separated chromatographically.

Compounds of formula (II) in which $R^3$ is an acyl group may themselves be produced by selectively hydrolysing a 3,3',5-triacylated derivative of a compound of formula (II), in the manner as described in Published European Patent Application No. 0 061 283.

The compounds of formulae (III) and (IV), are either known compounds, or can be prepared from known compounds by known methods.

The compounds of formula (I) may be formulated for use in a pharmaceutical composition. Accordingly, in a further aspect of the invention, there is provided a pharmaceutical composition which comprises a compound of the formula (I) together with a pharmaceutically acceptable carrier or excipient.

Compositions which may be administered by the oral route to humans may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more

usually 2 or 3 times a day. The effective dose of compound depends on the particular compound employed, but is in general in the range of from 1.0 to 20 mg/kg of body weight per day or more usually 2.0 to 10 mg/kg per day.

In a further aspect of the invention there is provided a method of treating viral infections in a human or non-human animal, which comprises administering to the animal an effective, non-toxic amount of a compound of formula (I).

The following Examples illustrate the invention.

Examples 1, 2 and 3

5-(E-2-Bromovinyl)-2'-deoxy-3',5'-bis-O-(tetrahydrofuran-2-yl)uridine (Example 1), 5-(E-2-bromovinyl)-2'-deoxy-3'-O-(tetrahydrofuran-2-yl)uridine (Example 2) and 5-(E-2-bromovinyl)-2'-deoxy-5'-O-(tetrahydrofuran-2-yl)uridine (Example 3).

To a sitrred solution of 5-(E-2-bromovinyl)-2'-deoxyuridine (1.32g) and 2-t-butoxytetrahydrofuran (1.0g) in tetrahydrofuran (10ml), a crystal of p-toluenesulphonic acid monohydrate was added and the mixture was stirred at room temperature for 5 min. It was then partitioned between aqueous sodium bicarbonate and ethyl acetate and the organic extract was dried and evaporated. The residue was chromatographed over silica gel (80g). Elution of the column with ethyl acetate-hexane (3:1) gave 5-(E-2-bromovinyl)-2'-deoxy-3',5'-bis-O-(tetrahydrofuran-2-yl)uridine (0.3g), νmax ($CHCl_3$) 1710, 1685, 1430 and 1280 $cm^{-1}$; $\delta_H$ ($CDCl_3$) 1.70-2.10 (8H, m), 2.10-2.55 (2H, m, 2'-$CH_2$), 3.40-4.40 (8H, m), 5.16 (2H, broad s), 6.18 (1H, m, 1'-CH), 6.64 (1H, s, J 14Hz, CH=CHBr), 7.35 (1H, d, J 14Hz, CH=CHBr), 7.80 (1H, s, 6-CH), 9.13 (1H, m, $D_2O$ exchangeable); and 5-(E-2-bromovinyl)-2'-deoxy-3'-O-(tetrahydrofuran-2-yl)uridine (0.32g), λmax (EtOH) 250 (ε 16,600) and 293 (ε 14,300) nm; νmax ($CHCl_3$) 1705, 1685, 1460, 1280 and 1100 $cm^{-1}$; $\delta_H$ [$(CD_3)_2SO$] 1.83 (4H, m), 2.20 (2H, m, 2'-$CH_2$), 3.50-4.40 (6H, m), 5.17 (1H, m), 5.20 (1H, m, $D_2O$ exchangeable, 5'OH), 6.13 (1H, m, 1'-CH), 6.88 (1H, d, J 14Hz, CH=CHBr), 7.33 (1H, d, J 14Hz, CH=CHBr), 7.86, 8.13 (1H, s, 6-CH), 11.66 (1H, broad s, $D_2O$ exchangable) (Found: C, 44.85; H, 4.3; N, 6.4%. $C_{15}H_{19}N_2O_6Br$ requires C, 44.7; H, 4.75; N, 6.95%); followed by 5-(E-2-bromovinyl)-2'-deoxy-5'-O-(tetrahydrofuran-2-yl)uridine (0.46g), m.p. 160-164°C (from ethyl acetate-hexane); λmax (EtOH) 251 (ε 14,100) and 293 (ε 12,100) nm; νmax (KBr) 1718, 1685, 1670, 1660 and 1280 $cm^{-1}$; $\delta_H$ [$(CD_3)_2SO$] 1.80 (4H, m), 2.13 (1H, t, J 6Hz, 2-$CH_2$), 3.40-4.25 (6H, m), 5.15 (1H, m), 5.30 (1H, m, $D_2O$ exchangeable, 3'-OH), 6.15 (1H, t, J 6Hz, 1'-CH), 6.86 (1H, d, J 14Hz, CH=CHBr), 7.30 (1H, d, J 14Hz, CH=CHBr), 7.85

(1H, s, 6-CH), 11.63 (1H, broad s, $D_2O$ exchangeable, NH) (Found: C, 44.55; H, 4.25; N, 6.75%. $C_{15}H_{19}N_2O_6Br$ requires C, 44.7; H, 4.75; N, 6.95%).

Examples 4, 5 and 6

5-(E-2-Bromovinyl)-2'-deoxy-3',5'-bis-O-(tetrahydropyran-2-yl)uridine (Example 4), 5-(E-2-bromovinyl)-2'-deoxy-3'-O-(tetrahydropyran-2-yl)uridine (Example 5) and 5-(E-2-bromovinyl)-2'-deoxy-5'-O-(tetrahydropyran-2-yl)uridine (Example 6)

A crystal of p-toluenesulphonic acid monohydrate was added to a stirred mixture of 5-(E-2-bromovinyl)-2'-deoxyuridine (2.08g) and dihydropyran (1.2ml) in tetrahydrofuran (30ml) at room temperature and the mixture was stirred at room temperature overnight. It was then partitioned between aqueous sodium bicarbonate and ethyl acetate. The organic extract was dried and evaporated and the residue was chromatographed over silica gel (70g). Elution of the column with ethyl acetate-hexane (4:1) afforded 5-(E-2-bromovinyl)-2'-deoxy-3',5'-bis-O-(tetrahydropyran-2-yl)-uridine (0.05g), $\nu$max ($CHCl_3$) 1700, 1680, 1460, 1278, 1124, 1070, 1030 and 905 $cm^{-1}$; $\delta_H$ ($CDCl_3$) 1.35-2.70 (14H, m), 3.35-4.85 (10H, m), 6.25 (1H, m, 1'-CH), 6.61, 6.73 (1H, d, J 14Hz, CH=CHBr), 7.34, 7.40 (1H, d, J 14Hz, CH=CHBr), 7.76, 7.94, 7.98 (1H, s, 6-CH), 9.37 (1H, m, $D_2O$ exchangeable, NH); followed by 5-(E-2-bromovinyl)-2'-deoxy-3'-O-(tetrahydropyran-2-yl)uridine (0.5g), $\lambda$max (EtOH) 250 ($\varepsilon$ 12,900) and 293 ($\varepsilon$ 11,000)nm; $\nu$max ($CHCl_3$) 1700, 1455, 1275, and 1100 $cm^{-1}$; $\delta_H$ ($CDCl_3$) 1.35-1.90 (6H, m), 2.05-2.70 (2H, m, 2'-$CH_2$), 2.60 (1H, m, $D_2O$ exchangeable, OH), 3.25-4.95 (7H, m), 6.15 (1H, t, J 6Hz, 1'-CH), 6.60 (1H, d, J 14Hz, CH=CHBr), 7.27 (1H, d, J 14Hz, CH=CHBr), 7.76, 7.80 (1H, s, 6-CH), and 9.10 (1H, m, $D_2O$ exchangeable, NH) (Found: C, 46.5; H, 5.05; N, 6.15%. $C_{16}H_{21}N_2O_6Br$ requires C, 46.05; H, 5.05; N, 6.7%); and 5-(E-2-bromovinyl)-2'-deoxy-5'-O-(tetrahydropyran-2-yl)uridine (0.5g), m.p. 160-162$^{o}$C (from acetone-ethyl acetate), $\lambda$max (EtOH) 250 ($\varepsilon$ 14,600) and 293 ($\varepsilon$ 12,500 nm; $\nu$max (KBr) 1685, 1470, 1385, and 1035 $cm^{-1}$; $\delta_H$ [$(CD_3)_2SO$] 1.30-1.82 (6H, m), 2.14 (2H, m, 2'-$CH_2$), 3.24-4.00 (5H, m), 4.20 (1H, m), 4.55 (1H, m), 5.24 (1H, m, $D_2O$ exchangeable, OH), 6.10 (1H, t, J 6Hz, 1'-CH), 6.76, 6.70 (1H, d, J 14Hz, CH=CHBr), 7.13, 7.15 (1H, d, J 14Hz, CH=CHBr), 7.75-7.85 (1H, s, 6-CH), and 11.53 (1H, m, $D_2O$ exchangeable, NH) (Found: C, 46.2; H, 4.7; N, 6.75%. $C_{16}H_{21}N_2O_6Br$ requires C, 46.05; H, 5.05; N, 6.7%).

Examples 7 and 8

5-(E-2-Bromovinyl)-2'-deoxy-3',5'-bis-O-(1-ethoxypropyl)-uridine (Example 7) and 5-(E-2-bromovinyl)-2'-deoxy-5'-O-(1-ethoxypropyl)uridine (Example 8)

A solution of 5-(E-2-bromovinyl)-2'-deoxyuridine (3.00g, 9mmol) in dry tetrahydrofuran (20ml) containing p-toluenesulphonic acid monohydrate (15mg) was charged with propionaldehyde diethyl acetal (5.90g, 7.5ml, 45mmol). The mixture was stirred at 25°C for 24 hours. The reaction mixture was poured onto saturated potassium hydrogen carbonate solution (50ml) and extracted with ethyl acetate (2 x 150ml). The organic phase was separated, dried ($MgSO_4$), and evaporated under reduced pressure. The crude products were subjected to short-path column chromatography using 10-30% acetone in n-hexane. Pure 5-(E-2-bromovinyl)-2'-deoxy-3',5'-bis-O-(1-ethoxypropyl)uridine was isolated as a glass (1.8g, 39% based on consumption of BVDU) with νmax (film) 1710, 1470, and 1288 $cm^{-1}$; $\delta_H$ ($CDCl_3$) 0.70-2.00 (16H, m, 4 x $CH_3$ and 2 x $CH_2$), 2.30-2.60 (2H, m, 2'-$CH_2$), 3.30-4.05 (6H, m, 5'-$CH_2$ and 2 x $CH_2O$), 4.15-4.75 (4H, m, 3'-CH, 4'-CH, 2 x CH), 6.10-6.50 (1H, m, 1'-CH), 6.75 (1H, dd, CH=CHBr), 7.45 (1H, d, CH=CHBr), 7.98 (1H, d, 6-CH), 9.63 (1H, br.s, $D_2O$ exchangeable, NH); m/z 504/506 ($M^+$, 1.5%), 401/403 ($M^+$-$C_5H_{11}O$, 6.5%); observed mass 401.0696, $C_{16}H_{22}N_2O_5Br$ requires 402.0712.

Pure 5-(E-2-bromovinyl)-2'-deoxy-5'-O-(1-ethoxypropyl)-uridine was isolated as a low melting foam (1.25g, 33% based on consumption of 5-(E-2-bromovinyl)-2'-deoxyuridine) with νmax (film) 1707, 1675, 1600, 1466, and 1280 $cm^{-1}$; $\delta_H$ [$(CD_3)_2SO$] 0.70-1.90 (8H, m, 2 x $CH_3$ and 1 x $CH_2$), 2.00-2.40 (2H, m, 2'-$CH_2$), 3.20-4.65 (7H, m, 3'-CH, 4'-CH, 5'-$CH_2$, $CH_2O$ and CH), 5.33 (1H, d, $D_2O$ exchangeable, OH), 6.00-6.40 (1H, m, 1'-CH), 6.83 (1H, d, CH=CHBr), 7.35 (1H, d, CH=CHBr), 7.95 (1H, d, 6-CH), 11.86 (1H, br.s, $D_2O$ exchangeable, NH); m/z 418/420 ($M^+$, 0.3%); observed mass 418.0724, $C_{16}H_{23}N_2O_6Br$ requires 418.0739.

0104857

Example 9

3',5'-Bis-O-benzoxymethyl-5-(E-2-bromovinyl)-3-p-chlorobenzoyl-2'-deoxyuridine

To a solution of 5-(E-2-bromovinyl)-3-p-chlorobenzoyl-2'-deoxyuridine (950mg, 2mmol) in dry N,N-dimethylformamide (20ml) was added diisopropylethylamine (5ml) and benzoxymethyl chloride (6.0ml, 6.60g) and the mixture stirred overnight at 25°C. The excess alkylating agent was removed by addition of a mixture of methanol (20ml) and pyridine (100ml). The solvents were removed under high vacuum and the residue subjected to short path column chromatography on silica gel using 10-20% chloroform in n-hexane as eluent. Pure 3',5'-bis-O-benzoxymethyl-5-(E-2-bromovinyl)-3-p-chlorobenzoyl-2'-deoxyuridine (1.35g, 94%) was obtained as an oil with νmax (film) 1750, 1700, 1664, 1590, 1450, 907 and 730 $cm^{-1}$; $\delta_H$ ($CDCl_3$) 1.90-2.50 (2H, m, 2'-$CH_2$), 3.65-3.90 (2H, m, 5'-$CH_2$), 4.00-4.90 (10H, m, 3'-CH, 4'-CH, 2 x $ArCH_2$ and 2 x $OCH_2$), 6.05-6.35 (1H, m, 1'-CH), 6.66 (1H, d, CH=CHBr), 7.10-7.80 (15H, m, CH=CHBr, $pClC_6H_4CO$ and 2 x $C_6H_5$), 7.90 (1H, s, 6-CH).

0104857

Example 10

3',5'-Bis-O-benzoxymethyl-5-(E-2-bromovinyl)-2'-deoxyuridine

A solution of 3',5'-bis-O-benzoxymethyl-5-(E-2-bromovinyl)-3-p-chloro benzoyl-2'-deoxyuridine (3.04g, 4.26mmol) in anhydrous tetrahydrofuran (200ml) at $0^oC$ was saturated with gaseous ammonia. The mixture was allowed to reach room temperature and stirred overnight. Removal of the tetrahydrofuran gave the crude reaction products. Short-path chromatography (silica gel, 10-20% acetone in n-hexane) failed to separate the desired product from p-chloroacetamide. Pure 3',5'-bis-O-benzoxymethyl-5-(E-2-bromovinyl)-2'-deoxyuridine was obtained (876mg, 36%) using silica gel spinning band chromatography (50:1 chloroform/methanol eluent) as a yellow oil with νmax (film) 1700, 1595, 1459, 1278, and 1020 $cm^{-1}$; $\delta_H$ [$(CD_3)_2SO$] 2.15-2.45 (2H, m, 2'-$CH_2$), 3.55-3.85 (2H, m, 5'-$CH_2$), 4.00-4.25 (1H, m, 4'-CH), 4.30-4.65 (3H, m, 3'-CH and $ArCH_2$), 4.80 (2H, d, $ArCH_2$), 6.15 (1H, t, 1'-CH), 6.83 (1H, d, CH=CHBr), 7.10-7.50 (11H, m, CH=CHBr and 2 x $C_6H_5$), 7.85 (1H, s, 6-CH), 11.50 (1H, br.s, NH, $D_2O$ exchangeable); m/z 573/575 ($MH^+$, 2%).

Examples 11 and 12

3'-O-Benzoxymethyl-5-(E-2-bromovinyl)-2'-deoxyuridine (Example 11) and 5'-O-benzoxymethyl-5-(E-2-bromovinyl)-2'-deoxyuridine (Example 12).

To a solution of 5-(E-2-bromovinyl)2'-deoxyuridine (2.00g, 6mmol) in tetrahydrofuran (20ml) and N,N-dimethylformamide (5ml) 2,4-lutidine (4.28g, 4.50ml, 40mmol) and benzoxymethyl chloride (3.14g, 3.00ml, 20mmol) were added. The resultant mixture was stirred at room temperature for 72 hours. The reaction mixture was poured into ice-water (100ml) and extracted with ethyl acetate (300ml). The organic phase was washed with dilute hydrochloric acid (100ml), saturated potassium hydrogen carbonate solution (300ml), water (200ml) and dried ($MgSO_4$). Removal of the solvent under reduced pressure followed by short path column chromatography(silica gel, 30% acetone in n-hexane) gave 3'-O-benzoxymethyl-5-(E-2-bromovinyl)-2'-deoxyuridine as an oil (0.21g, 7.7%) with νmax (film) 3400, 3175, 3050, 1700, 1460, 1280, 1100, and 1038 $cm^{-1}$; $\delta_H$ ($CDCl_3$) 1.60 (1H, br.s, 5'-OH, $D_2O$ exchangeable), 2.15-2.60 (2H, m, 2'-$CH_2$), 3.60-4.20 (3H, m, 5'-$CH_2$ and 4'-CH), 4.35-4.90 (5H, m, 3'-CH and 2 x $CH_2$), 6.20 (1H, t, 1'-CH), 6.63 (1H, d, CH=CHBr), 7.20-7.50 (6H, m, CH=CHBr and $C_6H_5$), 7.80 (1H, s, 6-CH), 8.90 (1H, br.s, NH, $D_2O$ exchangeable); MS (Positive Ion FAB) m/z 453 ($MH^+$, 2%).

Further elution (50% acetone in n-hexane) gave 5'-O-benzoxymethyl-5-(E-2-bromovinyl)-2'-deoxyuridine as a solid (0.325g, 12%) with m.p. 138-140$^oC$; νmax (film) 3400, 3240, 3060, 1700, 1675, 1598, 1468, 1278, and 1022 $cm^{-1}$; $\delta_H$ [$(CD_3)_2SO$] 2.00-2.40 (2H, m, 2'-$CH_2$), 3.50-4.05 (3H, m, 5'-$CH_2$ and 4'-CH), 4.10-4.43 (1H, m, 3'-CH), 4.70 (4H, d, 2 x $CH_2$), 5.18 (1H, d, 3'-OH, $D_2O$ exchangeable), 6.10-6.35 (1H, m, 1'-CH), 6.85 (1H, d, CH=CHBr), 7.10-7.50 (6H, m, CH=CHBr and $C_6H_5$), 7.95 (1H, s, 6-CH), 11.58 (1H, br.s, NH, $D_2O$ exchangeable).

Examples 13, 14 and 15

5-(E-2-Bromovinyl)-2'-deoxy-3',5'-bis-O-methoxymethyluridine (Example 13), 5-(E-2-bromovinyl)-2'-deoxy-3'-O-methoxymethyluridine (Example 14) and 5-(E-2-bromovinyl)-2'-deoxy-5'-O-methoxymethyluridine (Example 15)

To a solution of 5-(E-2-bromovinyl)-2'-deoxyuridine (3.00g, 9mmol) and dimethoxymethane (50ml) in anhydrous tetrahydrofuran (100ml) was added p-toluenesulphonic acid monohydrate (300mg) and the resultant mixture stirred at 25°. After 24 h the solution was poured into saturated sodium hydrogen carbonate solution (300ml). The aqueous mixture was extracted with ethyl acetate (250ml) and the organic phase washed with saturated sodium hydrogen carbonate solution (300ml), water (300ml) and dried (anhydrous magnesium sulphate). The ethyl acetate was removed in vacuo to give the crude reaction products. Short-path column chromatography (silica gel, 10-50% acetone in n-hexane) gave successively 5-(E-2-bromovinyl)-2'-deoxy-3',5'-bis-O-methoxymethyluridine (920mg, 24%) as a white solid with m.p. 101-103°; νmax (KBr) 1720, 1700, 1680, 1465, 1286, 1150, 1100, and 1032 $cm^{-1}$; $\delta_H$ ($CDCl_3$) 2.00-2.80 (2H, m, 2'-$CH_2$), 3.40 (6H, d, 2 x $CH_3O$), 3.60-4.00 (2H, m, 5'-$CH_2$), 4.10-4.45 (2H, m, 3'-CH and 4'-CH), 4.70 (4H, d, 2 x $CH_2O$), 6.25 (1H, t, 1'-CH), 6.63 (1H, d, CH=CHBr), 7.30 (1H, d, CH=CHBr), 7.87 (1H, s, 6-CH), 9.40 (1H, br.s, NH, $D_2O$ exchangeable); observed mass 420.0534, $C_{15}H_{21}N_2O_7Br$ requires 420.0533. 5-(E-2-bromovinyl)-2'-deoxy-3'-O-methoxymethyluridine (230mg, 6.80%) as a glass with νmax (film) 3400, 1705, 1467, 1280, amd 1027 $cm^{-1}$; $\delta_H$ [$(CD_3)_2SO$] 2.10-2.35 (2H, m, 2'-$CH_2$), 3.28 (3H, s, $CH_3O$), 3.40-3.70 (2H, m, 5'-$CH_2$), 3.80-4.00 (1H, m, 4'-CH), 4.15-4.40 (1H, m, 3'-CH), 4.65 (2H, s, $CH_2O$), 5.15 (1H, t, 5'-OH, $D_2O$ exchangeable), 6.10 (1H, m, 1'-CH), 6.80 (1H, d, CH=CHBr), 7.25 (1H, d, CH=CHBr), 8.05 (1H, s, 6-CH), 11.50 (1H, br.s, NH, $D_2O$ exchangeable); m/z 377/379 ($MH^+$, 4.00%). 5-(E-2-Bromovinyl)-2'-deoxy-5'-O-methoxymethyluridine (450mg, 13%) as a white solid with m.p. 173-175°; νmax (KBr) 3375, 3160, 1690, 1466, 1273, 1068, 1045, and 1027 $cm^{-1}$;

$\delta_H$ [$(CD_3)_2SO$] 2.05-2.35 (2H, m, 2'-$CH_2$), 3.30 (3H, s, $CH_3O$), 3.50-3.65 (2H, m, 5'-$CH_2$), 3.75-4.05 (1H, m, 4'-CH), 4.10-4.40 (1H, m, 3'-CH), 4.63 (2H, s, $CH_2O$), 5.30 (1H, d, 3'-OH, $D_2O$ exchangeable), 6.15 (1H, t, 1'-CH), 6.82 (1H, d, C$\underline{H}$=CHBr), 7.25 (1H, d, CH=C$\underline{H}$Br), 7.85 (1H, s, 6-CH), 11.50 (1H, br.s, NH, $D_2O$ exchangeable); observed mass 376.0272, $C_{13}H_{17}N_2O_6Br$ requires 376.0271.

Examples 16, 17 and 18

5-(E-2-Bromovinyl)-2'-deoxy-3',5'-bis-O-ethoxymethyluridine (Example 16), 5-(E-2-bromovinyl)-2'-deoxy-3'-O-ethoxymethyluridine (Example 17) and 5-(E-2-bromovinyl)-2'-deoxy-5'-O-ethoxymethyluridine (Example 18)

To a solution of 5-(E-2-bromovinyl)-2'-deoxyuridine (3.00g, ≈9mmol) in tetrahydrofuran (50ml) diethoxymethane (6.8ml, ≈54mmol) and p-toluenesulphonic acid monohydrate (500mg) were added. The resultant solution was stirred overnight at room temperature. The reaction mixture was poured into saturated potassium hydrogen carbonate solution (200ml) and the aqueous mixture extracted with ethyl acetate (2 x 200ml). The combined organic extracts were washed with saturated potassium hydrogen carbonate solution (2 x 200ml) and dried (magnesium sulphate). Removal of the ethyl acetate *in vacuo* gave the crude products. Short-path column chromatography on silica gel using 10% acetone in hexane as eluent gave 5-(E-2-bromovinyl)-2'-deoxy-3',5'-bis-O-ethoxymethyluridine (1.52g, 37% based on consumption of starting material) with νmax (film) 1700, and 1680 $cm^{-1}$; $\delta_H$ ($CDCl_3$) 1.25 (6H, m, 2 x $CH_3$), 1.90-2.65 (2H, m, 2'-$CH_2$), 3.45-4.00 (4H, m, 5'-$CH_2$ and 2 x $CH_3CH_2O$), 4.10-4.50 (2H, m, 3'-CH and 4'-CH), 4.80 (4H, m, 2 x $EtOCH_2O$), 6.25 (1H, m, 1'-CH), 6.65 (1H, d, CH=CHBr), 7.35 (1H, d, CH=CHBr), 7.90 (1H, s, 6-CH), 9.85 (1H, br.s, NH, $D_2O$ exchangeable); observed mass 448.0839, $C_{17}H_{25}N_2O_7Br$ requires 448.0846.

Subsequent elution with 25% acetone in n-hexane gave 5-(E-2-bromovinyl)-2'-deoxy-3'-O-ethoxymethyluridine (0.19g, 6.3% based on consumption of starting material) with νmax (film) 3440, 3175, 1700, 1100, and 1035 $cm^{-1}$; $\delta_H$ ($CDCl_3$) 1.20 (3H, t, $CH_3$), 2.10-2.60 (2H, m, 2'-$CH_2$), 2.83 (1H, br.s, 5'-OH, $D_2O$ exchangeable), 3.45-4.15 (5H, m, 4'-CH, 5'-$CH_2$ and $CH_3CH_2O$-), 4.23-4.52 (1H, m, 3'-CH), 4.75 (2H, s, $EtOCH_2O$-), 6.20 (1H, t, 1'-CH), 6.60 (1H, d, CH=CHBr), 7.28 (1H, d, CH=CHBr), 7.85 (1H, s, 6-CH), 9.50 (1H, br.s, NH, $D_2O$ exchangeable); observed mass 390.0430, $C_{14}H_{19}N_2O_6Br$ requires 390.0427.

0104857

Further elution with 30% acetone in *n*-hexane gave 5-(*E*-2-bromovinyl)-2'-deoxy-5'-O-ethoxymethyluridine (0.61g, 20% based on consumption of starting material) with νmax (film) 3425, 3225, 1710, and 1662 $cm^{-1}$; $\delta_H$ [$CDCl_3$ + 1 drop $(CD_3)_2SO$] 1.20 (3H, t, $CH_3$), 1.90-2.60 (2H, m, 2'-$CH_2$), 3.45-3.80 (4H, m, 5'-$CH_2$ and $CH_3C\underline{H}_2O$-), 4.05 (1H, m, 4'-CH), 4.30 (1H, m, 3'-CH), 4.75 (2H, s, $EtOC\underline{H}_2O$-), 5.00 (1H, d, 3'-OH, $D_2O$ exchangeable), 6.30 (1H, t, 1'-CH), 6.65 (1H, d, C<u>H</u>=CHBr), 7.28 (1H, d, CH=C<u>H</u>Br), 7.85 (1H, s, 6-CH), 10.85 (1H, br.s, NH, $D_2O$ exchangeable); observed mass 390.0422, $C_{14}H_{19}N_2O_6Br$ requires 390.0427.

Examples 19, 20 and 21

5-(E-2-Bromovinyl)-3',5'-bis-O-(2-chloro-1-ethoxyethyl)-2'-deoxyuridine (Example 19), 5-(E-2-bromovinyl)-3'-O-(2-chloro-1-ethoxyethyl)-2'-deoxyuridine (Example 20) and 5-(E-2-bromovinyl)-5'-O-(2-chloro-1-ethoxyethyl)-2'-deoxyuridine (Example 21)

To a solution of 5-(E-2-bromovinyl)-2'-deoxyuridine (3.33g, 10mmol) and chloroacetaldehyde diethylacetal (27.54g, 27ml, 180mmol) in anhydrous tetrahydrofuran (50ml) was added p-toluenesulponic acid monohydrate (1.5g) and the resultant mixture stirred at 25$^{o}$C. After 6 hours it was apparent (tlc) that no more starting material was being consumed and the reaction mixture was poured into saturated sodium hydrogen carbonate solution (300ml). The aqueous solution was extracted with ethyl acetate (300ml) and the organic layer washed with sodium hydrogen carbonate solution (300ml) and water (200ml) and dried (anhydrous magnesium sulphate). The ethyl acetate was removed in vacuo to give the crude reaction products. Short-path column chromatography (silica gel, 10-50% acetone in n-hexane) gave successively 5-(E-2-bromovinyl)-3',5'-bis-O-(2-chloro-1-ethoxyethyl)-2'-deoxyuridine (552mg, 10.10%) as an oil with νmax (film) 1706, 1055 $cm^{-1}$; $\delta_H$ ($CDCl_3$) 1.20 (6H, m, 2 x $CH_3$), 2.15-2.75 (2H, m, 2'-$CH_2$), 3.35-4.00 (10H, m, 5'-$CH_2$, 2 x $CH_2O$ and 2 x $ClCH_2$), 4.10-4.85 (4H, m, 4'-CH, 3'-CH and 2 x CH), 6.15-6.45 (1H, m, 1'-CH), 6.73 (1H, dd, CH=CHBr), 7.35 (1H, d, CH=CHBr), 7.80 (1H, d, 6-CH), 9.90 (1H, d, NH, $D_2O$ exchangeable); m/z 547 ($MH^+$, 21%). 5-(E-2-bromovinyl)-3'-O-(2-chloro-1-ethoxyethyl)-2'-deoxyuridine (294mg, 6.70%) as glass with νmax (film) 3450, 1706, 1468, 1286, 1110, and 1060 $cm^{-1}$; $\delta_H$ [$(CD_3)_2SO$] 1.15 (3H, t, $CH_3$), 2.25 (2H, m, 2'-$CH_2$), 3.40-3.75 (6H, m, 5'-$CH_2$, $ClCH_2$ and $OCH_2$), 3.90 (1H, m, 4'-CH), 4.35 (1H, m, 3'-CH), 4.73 (1H, t, CH), 5.15 (1H, t, 5'-OH, $D_2O$ exchangeable), 6.10 (1H, t, 1'-CH), 6.80 (1H, d, CH=CHBr), 7.23 (1H, d, CH=CHBr), 8.00 (1H, s, 6-CH), 11.50 (1H, s, NH, $D_2O$ exchangeable); m/z 439 ($MH^+$, 8.00%). 5-(E-2-Bromovinyl)-5'-O-(2-chloro-1-ethoxyethyl)-2'-deoxyuridine (736mg, 16.75%) as a foam with νmax (KBr) 3440,

1700, 1470, 1288, and 1060 $cm^{-1}$; $\delta_H$ [$(CD_3)_2$SO H, t, $CH_3$), 2.15 (2H, m, 2'-$CH_2$), 3.40-4.05 (7H, m, 5'-$CH_2$, $ClCH_2$, $OCH_2$ and 4'-CH), 4.25 (1H, m, 3'-CH), 4.75 (1H, t, CH), 5.33 (1H, d, 3'-OH, $D_2O$ exchangeable), 6.25 (1H, m, 1'-CH), 6.83 (1H, d, C<u>H</u>=CHBr), 7.25 (1H, d, CH=C<u>H</u>Br), 7.90 (1H, d, 6-CH), 11.50 (1H, s, NH, $D_2O$ exchangeable); m/z 439 ($MH^+$, 24%).

Example 22



5-(E-2-Bromovinyl)-2'-deoxy-3',5'-bis-O-α-methoxybenzyl-uridine

To a solution of 5-(E-2-bromovinyl)-2'-deoxyuridine (4.00g, 12mmol) in anhydrous tetrahydrofuran (20ml), p-toluenesulphonic acid monohydrate (0.005g) and benzaldehyde dimethyl acetal (11.00g, 11.00ml, 72mmol) were added and the resultant mixture stirred at room temperature for 72 hours. The reaction mixture was poured into saturated sodium hydrogen carbonate solution (100ml) and the aqueous slurry extracted with ethyl acetate (2 x 100ml). The organic extract was dried ($MgSO_4$) and evaporated under reduced pressure. Short-path column chromatography (silica gel, 10-30% acetone in n-hexane) gave 5-(E-2-bromovinyl)-2'-deoxy-3',5'-bis-O-α-methoxybenyluridine as a glass (2.65g, 38.5%) with νmax (film) 3180, 3060, 1700, 1455, 1280, 1100, 1060, 1030, 756, and 703 $cm^{-1}$; $\delta_H$ ($CDCl_3$) 1.70-2.75 (2H, m, 2'-$CH_2$), 3.20-3.90 (8H, m, 2 x $CH_3O$ and 5'-$CH_2$), 4.10-4.70 (2H, m, 3'-CH and 4'-CH), 5.30-5.65 (2H, m, 2 x ArCHOMe), 5.80-6.70 (2H, m, 1'-CH and CH=CHBr), 7.10-7.95 (12H, m, CH=CHBr, 6-CH and 2 x $C_6H_5$), 9.75 (1H, br.s, NH, $D_2O$ exchangeable) (Found: C, 56.64; H, 4.87; N, 4.77%. $C_{27}H_{29}N_2O_7Br$ requires C, 56.54; H, 5.06; N, 4.88%).

## Antiviral Activity

### In Vitro

### Method

Vero (African Green Monkey Kidney) cells were grown to confluence in 24 well multidishes, each well being 1.6 cm in diameter. The cells were infected with Herpes simplex type 1 virus (HFEM strain) and overlaid with 0.5ml of 0.9% agarose (w/v) in maintenance medium. Test compounds prepared in maintenance medium in concentrations ranging from 100 to 0.03 µg/ml in half-log dilution steps, were added in 0.5ml volume. The virus infected cultures were then incubated at 37°C for 4 days before fixing in 4% formaldehyde solution and staining with carbolfuchsin. The dishes were then examined to find what concentration of test compound caused a 50% reduction in the number of virus plaques formed ($PDD_{50}$ value) and the minimum concentration of test compound which caused cytotoxicity (MTD).

### Results

| Example No | $PDD_{50}$ µg/ml | $PDD_{50}$ µM | MTD (µg/ml) |
|---|---|---|---|
| 1 | 4.3 | 9.0 | >100 |
| 2 | 1.1 | 2.8 | >100 |
| 4 | 17 | 34 | 100 |
| 5 | 1.8 | 4.3 | >100 |
| 6 | 69 | 170 | >100 |
| 8 | 6.4 | 15 | >100 |
| 10 | 72 | 126 | >100 |
| 12 | 6.6 | 15 | >100 |
| 19 | 9.1 | 17 | >100 |
| 20 | 3.3 | 7.5 | >100 |
| 21 | 19 | 43 | >100 |

## CLAIMS

1. A compound of formula (I):

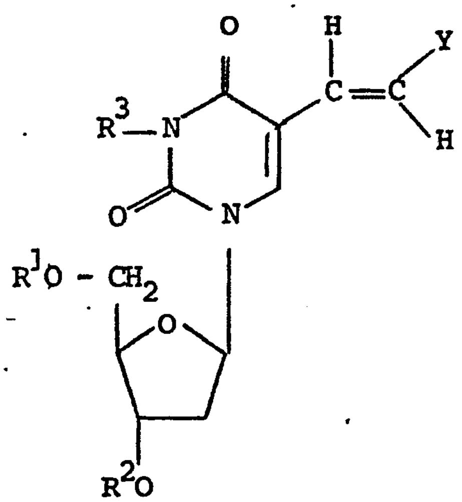

(I)

in which Y is chlorine, bromine or iodine, each of $R^1$ and $R^2$ is hydrogen or a group of formula $R^4-\underset{|\atop OR^5}{CH}-$ in which $R^4$ is hydrogen, optionally halo-substituted $C_{1-6}$ alkyl, phenyl or phenyl - $C_{1-6}$ alkyl, $R^5$ is optionally halo-substituted $C_{1-6}$ alkyl or phenyl-$C_{1-6}$alkyl, or $R^4$ and $R^5$ complete a ring system and together represent $-(CH_2)_3-$, $-(CH_2)_4-$ or $-CH_2OCH_2CH_2-$,

and $R^3$ is hydrogen or an acyl group of formula

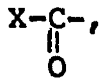

in which X is $C_{1-6}$alkyl, phenyl or phenyl-$C_{1-6}$alkyl, each of which is optionally substituted with halogen, with the proviso that $R^1$ and $R^2$ are not simultaneously hydrogen.

2. A compound according to claim 1, in which $R^2$ is hydrogen.

3. A compound according to claim 1 or claim 2, in which $R^4$ is hydrogen, methyl, chloromethyl, ethyl or phenyl, and $R^5$ is methyl, ethyl or benzyl.

4. A compound according to claim 1 or claim 2, in which $R^4$ and $R^5$ together represent $-(CH_2)_4-$.

5. A compound according to any one of claims 1 to 4 in which Y is bromine.

6. A compound according to claim 1 selected from
5-(E-2-bromovinyl)-2'-deoxy-5'-0-(tetrahydrofuran-2-yl)uridine;
5-(E-2-bromovinyl-2'-deoxy-5'-0-(tetrahydropyran-2-yl)uridine;
5-(E-2-bromovinyl)-2'-deoxy-5'-0-(1-ethoxypropyl)-uridine;
5'-0-benzoxymethyl-5-(E-2-bromovinyl)-2'deoxyuridine;
5-(E-2-bromovinyl)-2'-deoxy-5'-0-methoxymethyl-uridine;
5-(E-2-bromovinyl)-2'-deoxy-5'-0-ethoxymethyl-uridine;
5-(E-2-bromovinyl)-5'-0-(2-chloro-1-ethoxyethyl)-2'-deoxyuridine;

7. A process for preparing a compound of formula (I), as defined in claim 1, which comprises

a) treating a compound of formula (II):

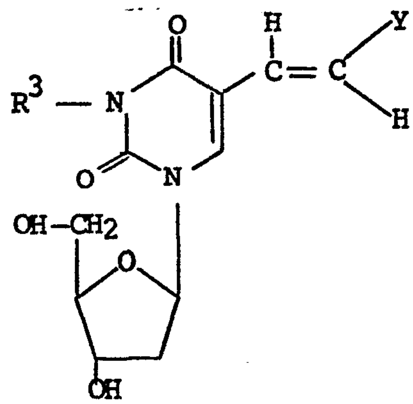

(II)

in which Y and $R^3$ are as defined in formula (I), with an ether of formula (III):

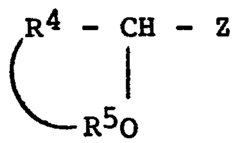

(III)

in which Z is hydrogen or a $C_{1-6}$ alkoxy group, and, when Z is hydrogen, the $-R^4-R^5-$ group includes a double bond adjacent to the oxygen, or

b) treating a compound of formula (II) with a compound of formula (IV):

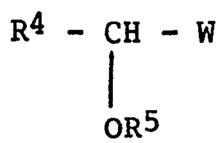

(IV)

in which W is a $C_{1-6}$ alkoxy group or a halogen atom.

8. A pharmaceutical composition which comprises a compound of formula (I), as defined in claim 1, together with a pharmaceutically acceptable carrier or excipient.

9. A composition according to claim 8, in unit dosage form.

10. A compound according to any one of claims 1 to 6, or a composition according to claim 8 or 9, for use in treating viral infections.

0104857

European Patent Office

# EUROPEAN SEARCH REPORT

Application number

EP 83 30 5481

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-3 010 399 (GAURI K.K.) * Pages 5-7 * | 1,8 | C 07 H 19/06<br>A 61 K 91/70 |
| Y | DE-A-2 915 254 (UNIVERSITY OF BIRMINGHAM (GB)) * Pages 3,4 * | 1,8 | |
| Y | CHEMICAL ABSTRACTS, vol. 94, no. 11, 16th March 1981, page 758, no. 84422r, Columbus, Ohio, US I.D. CHKANIKOV et al.: "Synthesis of glycoside and o-tetrahydrofuranyl derivatives of nucleosides" & AKTUAL'N. PROBL. EKSPERIM. KHIMIOTERAPII OPUKHOLEI. MATERIALY VSES. SOVESHCH., CHERNOGOLOVKA 1980, 1, 51-54 * Abstract * | 1,8 | TECHNICAL FIELDS SEARCHED (Int. Cl. 3) |
| Y,D P | EP-A-0 061 283 (BEECHAM) * Pages 1-3 * | 1,8 | C 07 H 19/00<br>A 61 K 31/00 |

The present search report has been drawn up for all claims

| Place of search | Date of completion of the search | Examiner |
|---|---|---|
| THE HAGUE | 10-11-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82